(19) Europäisches Patentamt
European Patent Office
Office européen des brevets

(11) Publication number: **0 247 538 A2**

# (12) EUROPEAN PATENT APPLICATION

(21) Application number: **87107494.4**

(22) Date of filing: **22.05.87**

(51) Int. Cl.⁴: **C07K 3/02**, C07K 15/06, C07K 1/14, A61K 37/02

(30) Priority: **28.05.86 US 867564**

(43) Date of publication of application: **02.12.87 Bulletin 87/49**

(84) Designated Contracting States: **BE CH DE FR GB IT LI LU NL SE**

(71) Applicant: **Imreg, Inc.**
**Suite 1400, 144 Elk Place**
**New Orleans, LA 70112(US)**

(72) Inventor: **Gottlieb, Arthur A.**
**c/o IMREG, INC. 144 Elk Place Suite 1400**
**New Orleans Louisiana 70112(US)**
Inventor: **Sizemore, Robert C.**
**c/o IMREG, INC. 144 Elk Place Suite 1400**
**New Orleans Louisiana 70112(US)**
Inventor: **Sinha, Sudhir K.**
**c/o IMREG, INC. 144 Elk Place Suite 1400**
**New Orleans Louisiana 70112(US)**

(74) Representative: **Popp, Eugen, Dr. et al**
**MEISSNER, BOLTE & PARTNER**
**Widenmayerstrasse 48 Postfach 86 06 24**
**D-8000 München 86(DE)**

## (54) Immunosuppressor and method of extraction thereof.

(57) A method of extracting a suppressor, herein designated L-4, from an extract of leukocytes and purifying it whereby said suppressor is separated from extraneous materials in said extract, comprising the following steps:

(1) size fractionating said extract, thereby producing a first dialysate having a nominal M.W. cutoff of approximately 12,000;

(2) size fractionating said first dialysate, thereby producing a seond dialysate and a retentate, separated from one another at a nominal M.W. cutoff of approximately 3500;

(3) applying said retentate to a reverse phase HPLC column;

(4) eluting said column with an acetonitrile in dilute aqueous trifluoroacetic acid gradient, said gradient including a range of acetonitrile concentration from approximately 70% to approximately 80%, thereby producing elutants associated with different portions of said gradient; and

(5) collecting the elutant associated with the zone of said gradient wherein acetonitrile concentration is between approximately 75% and approximately 80%, and a product of such a process.

EP 0 247 538 A2

# IMMUNOSUPPRESSOR AND METHOD OF EXTRACTION THEREOF

## BACKGROUND OF THE INVENTION

This invention concerns cell-mediated immunity. A typical manifestation of cell-mediated immunity is the delayed hypersensitivity ("DH") skin reaction, which is observed when an appropriate antigen is injected subcutaneously. Within 24 to 48 hours, local inflammation (erythema) and a swelling and thickening (induration) are observed in a sensitive individual. The degree of sensitivity may be measured by the size and severity of the reaction. The DH reaction also presents characteristic histological findings --specifically, perivascular infiltration of lymphocytes and monocytes in the inflamed area. The cells seen at the site of a DH reaction are derived from the peripheral blood leukocyte (PBL) population. The mechanisms of cell-mediated immunity are as yet incompletely understood. It is known that the cells which mediate the response are capable of responding in a variety of ways to a challenge from an antigen. These responses include: proliferation of cells bearing specific sensitivity to a given antigen; the induction and multiplication of cells mediating a variety of immune functions, including antibody production; and reactions against foreign cells (such as pathogens or transplants) and tumors.

The present invention relates to the discovery of (1) a method for extracting a "suppressor" of the immunity system, which is isolated from dialyzed extracts of leukocytes, and (2) the suppressor itself that is so extracted. It has further been discov ered that this suppressor profoundly affects the quality and quantity of cell-mediated immunity responses; is potentially useful in the treatment of a variety of clinical conditions characterized by excessive or undesired reaction to a specific cell or antigen; and is potentially useful in the alleviation of certain allergic conditions.

The term "suppressor," and much of the background relevant to the instant application, is explained in Gottlieb U.S. Patent No. 4,468,379 (hereinafter referred to as the '379 patent), August 28, 1984; and EPO Pub. No. 0 042 064, 23.12.81. The prior art in this field is also discussed in said references. That discussion is incorporated herein by reference. For the present purpose, the term suppressor may be considered to be a substance that, when administered to a human or other mammalian subject, causes a lessening of the magnitude or rate of immune system reaction, for example as measured by DH reaction.

## SUMMARY OF THE PRESENT INVENTION

The instant invention concerns a process for the extraction of a suppressor, not hitherto described, which is hereinafter designated as L-4, and the discovery of suppressor material that can be extracted from leukocyte preparations by said process. A chromatography system of the instant invention uses acetonitrile-trifluoroacetic acid aqueous gradients, and permits the isolation of suppressor material. Methods of using L-4 and related products are described, as are pharmaceutical compositions containing such products.

## DETAILED DESCRIPTION OF THE PREFERRED EMBODIMENT

In the following discussion, procedures are described wherein materials were obtained from human donors and test measurements were made on human recipients. The procedures and reagents used herein were chosen to provide sterile and non-toxic products for human treatment. Despite the toxicity of the solvent acetonitrile ($CH_3CN$), which is used, the end products of the procedures are nontoxic and they are free of acetonitrile.

### Extraction Procedure

The initial step in the preparation of the modulator material of this invention is preparation of leukocyte pellets, followed by separation of the fractions of leukocyte extract of interest herein, viz., those with M.W. between 3500 and 12,000.

EXAMPLE 1 --Preparation and Filtration of Leukocyte Extracts

Leukocyte pellets were prepared by the methods of Example 1 of the '379 patent. "L" (or "large" M.W.) fraction of the extract is isolated by passing the material through a filter having a nominal 12,000 M.W. cutoff. The material passed through is then cut off at M.W. >3500 by ultrafiltration or by dialysis in accordance with Example 2 of the '379 patent.

The material with 3500 < M.W. < 12,000 is then dried, reconstituted in 0.1% aqueous trifluoroacetic acid, and set aside for the next step.

EXAMPLE 2 --Reverse Phase Liquid Chromatography

Further purification of the material of preceding Example 1 was carried out by reverse-phase high pressure liquid chromatography ("HPLC") on octadecylsilane (ODS). A Novapack C-18 3.9 mm x 15 cm column was used with a Waters 680 Series Liquid Chromatograph, but an equivalent instrument made by another manufacturer may be used. The following solvent and gradient system was used, all flow rates 1.0 ml/min:

Solvent A = 80% acetonitrile in 0.1% aqueous solution of trifluoroacetic acid
Solvent B = 0.1% trifluoroacetic acid, pH 2

| Time | %A | %B |
|---|---|---|
| 0 | 10 | 90 |
| 20 | 100 | 0 |
| 30 | 100 | 0 |
| 35 | 10 | 90 |

A fraction elutes at 22.0 to 26.0 minutes, which is designated hereinafter as L-4. It may be recognized by its UV absorption characteristics, A 214, Full Scale (F.S.) = 0.5 A.U. (Absorbance Units), which are as follows: During the first 3 to 6 minutes a small number of narrow, high peaks appear, which are not of interest herein. From about 5 to 7 minutes there is a deep trough. At approximately 7 through 15 minutes there is a high, broad peak, which is also not of interest herein except as a marker for L-4, which follows shortly. The next high (approximately 0.1 F.S.) peak occurs at approximately 24 minutes; it is very narrow (less than 1 minute). Subsequent assay shows suppressor activity of this fraction, which is designated L-4. The material is dried and stored at -70°C.

**Nature of Material of Interest**

The foregoing HPLC data makes it possible to characterize the L-4 material of interest with sufficient particularity to permit repeatable extractions. However, it is not possible as yet to characterize the structure of the molecule or molecules of interest in terms of a chemical formula. Certain physical and chemical features can be inferred, however, from the data.

First, there may be some peptide material present in the molecule or molecules of interest, because the particular UV absorption is typically associated with a peptide bond. The molecule or molecules of interest may be polypeptides with other groups attached. It is uncertain how many peptide groups are involved. The fact that 3500 < M.W. < 12,000 indicates the possibility of considerably more peptide groups than the two or three groups present in the inventor Gottlieb's co-pending EPO PATENT APPLICATION FOR IMMUNOAMPLIFIERS, No. 86 118 104.8.

Second, the L-4 material comes off the column in the zone of approximately 80% ± 5% acetonitrile concentration. That indicates that the molecule or molecules of interest are relatively hydrophobic, which in turn suggests that the molecule or molecules of interest have a high affinity for a relatively waxy column and probably contain a considerable C or CH component, such as several groups such as $CH_3$, $C_2H_5$, acetyl,

COOH, glucose. Because of the high weight, ring groups should not be excluded from consideration. Of course, since this is a product produced in the human body, the groups must be nontoxic, pharmaceutically acceptable ones or the human body could never have evolved L-4 as a messenger material for maintaining homeostasis in the human immune system.

Based on general information about lymphokines and like biological agents, it is considered that the molecule or molecules of interest may be essentially polypeptides with added organic constituents attached to at least some of the peptide groups. A possible structure is:

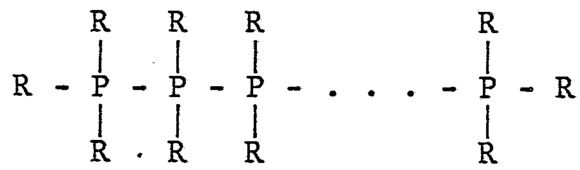

where P is an amino acid group such as Tyr, Gly, etc., and where R is a pharmaceutically acceptable organic group such as acetyl, ethyl, etc. (In the diagram, the different P's may be different amino groups and the different R's may be either nulls [i.e., no substitution] or different aforesaid organic groups.) As used hereinafter, the term "essentially consisting of polypeptide material" is intended to refer to molecules like that depicted above, e.g., acetylated and/or methylated and/or ethylated and/or esterified polypeptides.

Further, the foregoing information suggests that it would be appropriate to methylate, ethylate, acetylate, etc. the natural polypeptide material, using known organic chemistry techniques, in order to determine whether the resulting product has improved pharmacological action. It is known that use of molecular modifi cations of this type may improve blood level, speed of absorption, and other pharmacological parameters. Examples in the literature include the deschlorination of chlortetracycline to produce tetracycline, and the saturation of a carbon bond in chlorothiazide to produce hydrochlorothiazide.

**Demonstration of Suppressor Activity**

The L-4 material of Example 2 is tested for suppressor activity by measuring its ability to reverse antigen-induced migration inhibition in a direct leukocyte or macrophage migration inhibition assay. A sample of the L-4 material of Example 2 was reconstituted with sterile saline and is successively diluted with medium to provide the following preparations:

| Preparation | A | B | C | D | E | F |
|---|---|---|---|---|---|---|
| Dilution (1:__) | 100 | 500 | 1000 | 5000 | 10,000 | 20,000 |

The direct leukocyte migration inhibition (LMI) assay is an *in vitro* correlate of delayed type hypersensitivity (DH). The LMI assay examines the effect of leukocyte migration inhibitory factor (LIF) on migrating polymorphonuclear cells. The assay is carried out with triplicate samples (if possible; otherwise, duplicate) for each of the above dilutions with and without antigen; in addition, there are controls without and without antigen, which receive no L-4. (Since there are 6 dilutions and 1 control, the total for the triplicate procedure with antigen and without antigen is 3 x 7 x 2 = 42.)

EXAMPLE 3 --LMI assay of L-4

a. Preparation of pellets

A gelatin solution of 2% Knox Gelatin in 0.9% NaCl was prepared by heating in a boiling water bath for 10-15 min. The solution was then equilibrated to 37°C.

Whole heparinized peripheral blood from a tetanus toxoid (TT) or PPD sensitive donor was diluted 1:1 (v/v) with the gelatin solution in 50 cc polypropylene tubes and mixed. The tubes were then incubated in a 37°C incubator for 20 min at a 30° slant and then 10 min upright.

The leukocyte-containing supernatant was transferred to 50 cc polypropylene tubes and centrifuged for 10 min at 1200 rpm, 25°C, to pellet cells.

Each pellet was resuspended in approximately 30 ml Tris-$NH_4Cl$ lysing buffer preequilibrated to 37°C; the suspension was then incubated at 37°C for 10 min. In several instances, it was necessary to repeat the lysing-incubation step, by combining two pellets in 35 ml of the buffer.

Pellets were pooled in a 15 ml conical tube and were washed twice with cold RPMI-1640 (GIBCO) + 10% FCS. The material was then centrifuged for 10 min at 1200 rpm, 10°C, to pellet the cells.

b. Preparation of hematocrit tubes

The pellets were resuspended to 4 x $10^7$/ml in RPMI-1640 + 10% FCS + 2% PSN (Complete RPMI). The resulting cell suspension was placed in microhematocrit capillary tubes (75 µl) (as used herein, µl = microliter, µg = micrograms), by capillary action. The tubes were plugged with clay (Seal-Ease), which was gently tapped to insure a good seal. The tubes were placed on ice.

The tubes were centrifuged at approximately 800 rpm for 10 min, 10°C.

c. Addition of reagents

Enough tubes were selected to permit triplicate samples for each group in the assay. The assay involves 42 samples for triplicate, or if only duplicate samples can be provided, 28. The control group is Complete RPMI without other reagents.

Each tube was scored with an ampule file at the cell-fluid interface. The tubes were gently broken and positioned in the wells of a 24-well Costar plate, after greasing the plug ends with silicone grease. Each tube was perpendicular to and in contact with the well wall.

Each tube was overlaid with 1 ml Complete RPMI. For the tubes to which antigen was to be added, TT pr PPD was also added (TT = 100-150 Lf/ml, PPD = 75-100 µg/ml). For the tubes to which L-4 was to be added, the appropriate dilution was also added.

The plates were incubated for 16-18 hours at 37°C to permit leukocyte migration.

d. Measurements and calculations

The plates were each placed on an overhead projector and the migration patterns were projected onto paper, where they were traced. For each fan, two perpendicular diameters were measured. The migration area was calculated for each fan, and groups within the collection were averaged.

The **percent migration inhibition (PMI)** is defined in terms of the foregoing data. The following parameters are defined in terms of average fan areas (AFA):

A = AFA for culture of antigen + particular L-4 dilution
B = AFA for culture of particular L-4 dilution
C = AFA for culture of antigen
D = AFA for culture of Complete RPMI alone.

The PMI is then defined as:

Experimental PMI = 100[1 - (A/B)]
Control PMI = 100[1 - (C/D)].

The following percentages of migration inhibition (Table A) were observed for the various preparations:

Table A - PMIs for Various L-4 Dilutions - LIF Assay

| Preparation | PMI (%) |
|---|---|
| A | 48.69 ± 9.4 |
| B | 21.60 ± 5.8 |
| C | 34.79 ± 1.9 |

| | |
|---|---|
| D | 9.32 ± 8.2 |
| E | 46.64 ± 6.4 |
| F | 29.53 ± 6.3 |
| Control | 43.09 ± 2.8 |

The data indicates that addition of L-4 to a direct leukocyte migration inhibition assay resulted in reversal of antigen-induced migration inhibition in a dose-dependent manner. Although the effect depended on concentrations of reagents, it was established that L-4 had no significant effect on leukocyte migration in the absence of antigen. Thus the L-4 suppressed (i.e., reversed) antigen-induced LIF activity.

In addition, an assay was made by measuring **macrophage migration inhibition (MMI),** using peripheral blood monocytes as indicator cells. The same preparations were used as in Example 3, except that Preparation A was not used.

EXAMPLE 4 - MMI assay of L-4

Mononuclear cells were prepared by standard Ficoll-Hypaque density centrifugation of whole blood from an antigen-sensitive donor. To assay 5 dilutions of a test sample, approximately 200-250 ml blood is necessary.

Except for the omission of gelatin solution, pellets were prepared as in Example 3 - § a.

Hematocrit tubes were prepared as in Example 3 - § b, except that the initial resuspension of pellet was to 1 x $10^8$/ml. The procedures of Example 3 - §§ c and d were then followed (without Preparation A).

The PMI data corresponding to that of Example 3 is shown below in Table B.

Table B - PMIs for Various L-4 Dilutions - MMI Assay

| Preparation | PMIF (%) |
|---|---|
| B | 28.23 ± 10.74 |
| C | 21.30 ± 3.49 |
| D | 30.41 ± 3.90 |
| E | 31.49 ± 8.77 |
| F | 37.91 ± 2.66 |
| Control | 39.15 ± 3.90 |

The data indicates that addition of L-4 to this system also resulted in a dose-dependent reversal of antigen-induced migration inhibition. As in the LMI assay, L-4 had no significant effect in the absence of antigen. Thus, L-4 reversed macrophage inhibition factor (MIF) as well as LIF activity.

It is thus seen that L-4 is a potent immunosuppressor and anti-inflammatory agent, and its use is indicated for conditions in which suppression of immune response is sought, such as in preparing patients to receive organ transplants, contact dermatitis (e.g., from poison ivy or other catechol-containing products), and other hyperimmune or autoimmune conditions (e.g., rheumatoid arthritis, lupus, diabetes type I). It may appropriately be administered for this purpose by injection for systemic effects and topically or subcutaneously for local effect.

EXAMPLE 5 - Poison ivy treatment

A patient suffers from erythema and itching resulting from poison ivy. A salve is prepared in a cold cream base, containing a medically predetermined amount (e.g., from approximately 1 ng/ml to approximately 2 ng/ml) of L-4. The salve is applied to the affected skin area every four hours until erythema subsides.

EXAMPLE 6 - Rheumatoid arthritis

A patient suffers from rheumatoid arthritis, which is believed to have a significant autoimmune component. the patient is given weekly subcutaneous or intradermal injections of 0.1 ml of a preparation containing a medically predetermined amount (e.g., for approximately 100 pg/ml to approximately 200 pg/ml) of L-4 until joint inflammation subsides. After that, the same injections are given biweekly.

EXAMPLE 7 - Lupus

A patient suffers from lupus erythematosis. The patient is given weekly subcutaneous or intradermal injections of 1 ml of a preparation containing an amount of L-4 that the attending physician considers medically appropriate (e.g., approximately 10 pg/ml to approximately 20 pg/ml) for 6 weeks. The attending physician monitors the patient's condition and increases or decreases the dose in accordance with the physician's medical judgment.

**GENERAL CONCLUDING REMARKS**

The above described procedures disclose what the inventor believes is a hitherto unknown method of modifying human immune system response. The disclosure also describes hitherto unknown compositions for effecting such immunomodulation, as well as novel extraction procedures for obtaining the desired material in medically (pharmaceutically) acceptable form.

While the invention has been described primarily in connection with a specific and preferred embodiment thereof, it will be understood that it is capable of further modifications without departing from the spirit and scope of the invention. This application is intended to cover all variations, uses, or adaptations of the invention, following, in general, the principles of the invention and including such departures from the present disclosure as come within known or customary practice within the art to which the invention pertains, or as are obvious to persons skilled in the art, at the time the departure is made.

As used in the claims, the terms **dialyze, dialysate,** and related words are intended to comprehend equivalent means of separating molecules and the material resulting from such separation, such as by use of ultrafiltration, ultracentrifugation, electrophoresis, and the like. Thus **dialysate** includes ultrafiltrate. The term **size fractionating** includes dialyzing, ultrafiltering, and doing like processes.

**Dilute aqueous trifluoroacetic acid** solution means approximately 0.1% solution.

**Chromatogtraphically elutable** with a specified solvent and gradient system has the meaning indicated in the '379 patent, column 15. That is, generally speaking, having the property of being able to be eluted by reverse phase HPLC with the specified solvent, usually as specified with greater particularity in terms of the gradient zone in which the material elutes, the particular gradient zone being designated in terms of solvent concentration, refractive index, UV absorption, and/or other characteristics that would permit a person skilled in the art to recognize the zone in question and thus known which elutant to collect. The matrix (such as ODS) may also be specified.

When reference is made to UV absorption profiles, the wavelength in question is approximately 214 nm.

The term **vehicle** is intended to include the term **carrier.**

As indicated in the section concerning the nature of the material of interest, following Example 2, supra, the L-4 material described herein may be a single molecular species or a mixture of molecular species. The UV absorption characteristics suggest presence of one or more peptide bonds. Hence, L-4 material may be a polypeptide present in one or more isomeric forms, and present as one or more derivatives of such forms (e.g., esters, amides, acetylated polypeptides). The procedure developed thus far does mot permit the inventor to give detailed information about the molecular structure of L-4. However, the available information permits the description of a material that can be reproducibly produced by means of the procedures described in the specification.

Also, the UV profile data associated with the product permits an analytic test of a material to determine whether it is L-4. That is, it is possible to take a material and determine whether it is L-4 by means of the procedure of Example 2, supra. The material is placed in the HPLC system of Example 2, where it may be accompanied by a tracer. If the material of interest elutes at approximately 24 minutes under the HPLC conditions stated in Ex ample 2 (approximately 80% concentration of acetonitrile) and is accompanied by a UV absorption peak of appropriate magnitude, the material is probably L-4. The fact can be confirmed by assay procedures such as those of Example 3, supra.

Accordingly, it is considered that the scope of the invention includes not only the novel extraction procedures described in the specification, but also the peptide material herein designated as L-4. In addition, the invention is considered to include not only the naturally occuring L-4 material, but the same material when methylated, ethylated, acetylated, etc. to produce non-natural but pharmaceutically acceptable alternative molecular species, as described above in the specification preceding Example 3.

## Claims

1. A method of extracting a suppressor, herein designated L-4, from an extract of leukocytes and purifying it whereby said suppressor is separated from extraneous materials in said extract, comprising the following steps:

(1) size fractionating said extract, thereby producing a first dialysate having a nominal M.W. cutoff of approximately 12,000;

(2) size fractionating said first dialysate, thereby producing a seond dialysate and a retentate, separated from one another at a nominal M.W. cutoff of approximately 3500;

(3) applying said retentate to a reverse phase HPLC column;

(4) eluting said column with an acetonitrile in dilute aqueous trifluoroacetic acid gradient, said gradient including a range of acetonitrile concentration from approximately 70% to approximately 80%, thereby producing elutants associated with different portions of said gradient; and

(5) collecting the elutant associated with the zone of said gradient wherein acetonitrile concentration is between approximately 75% and approximately 80%.

2. The process of claim 1 wherein the elutant collected is further characterized in terms of its UV absorption as being associated with a high, narrow peak which is the highest peak following a broad, high peak, which follows a deep trough.

3. The product of the process of claim 1 or claim 2.

4. A suppressor material having a M.W. between 3500 and 12,000, and further characterized by the properties of being:

(a) substantially entirely chromotographically elutable by acetonitrile in dilute aqueous trifluoroacetic acid gradient, in the portions of said gradient between approximately 70% and approximately 80% acetonitrile concentration;

(b) substantially not so elutable in the portions of said gradient below approximately 70% acetonitrile concentration; and

(c) substantially free of material that is so elutable below said concentration and of material that is so elutable only above 90% acetonitrile concentration.

5. Material of claim 4 further characterized by the presence of at least one peptide bond and by the absence of pharmaceutically unacceptable contaminants.

6. Material of claim 5 essentially consisting of polypeptide material.

7. Material of claim 4 further characterized by being associated --when said material is chromatographically eluted from a dialysed leukocyte extract with a chromatography system comprising an acetonitrile-in-dilute-aqueous-trifluoroacetic-acid gradient, and the elutant of said chromatographic system is monitored for UV absorption --with a high narrow UV absorption peak which is the highest peak following a broad high peak which follows a deep trough.

8. The composition of matter of claim 5, in a pharmaceutically acceptable vehicle and in an effective dosage amount.

9. A method of decreasing the speed or magnitude of a person's immune system response comprising administration to said person of the composition of matter of claim 4.

10. A method of treating a person for an automimmune or hyperimmune condition comprising administration to said person of the composition of matter of claim 4.

11. The method of claim 10 wherein said person suffers from rheumatoid arthritis, diabetes type I, or systemic lupus erythematosus.

12. The method of claim 9 wherein said person suffers from contact dermatitis.

13. The method of claim 9 wherein said person requires an organ transplant.

14. The composition of matter of claim 5 combined with at least one pharmaceutically acceptable organic group having 1-6 carbon atoms.

15. The composition of matter of claim 14 wherein said group is methyl, ethyl, or acetyl.